# EUROPÄISCHE PATENTSCHRIFT

(11) **EP 1 399 437 B1**
(45) Veröffentlichungstag und Bekanntmachung des Hinweises auf die Patenterteilung: **26.01.2005**
(21) Anmeldenummer: 02738069.0
(22) Anmeldetag: 14.05.2002
(51) Int. Cl.: C07D 405/06

(54) **VEFAHREN ZUR HERSTELLUNG VIN TRIAZOLYLMETHYL-OXIRANEN**
METHOD FOR THE PRODUCTION OF TRIAZOLYLMETHYLEPOXIDES
PROCEDE DE PRODUCTION DE TRIAZOLYLMETHYLE-OXIRANNES

(30) Priorität: 18.05.2001 DE 10124666
(43) Veröffentlichungstag der Anmeldung: 24.03.2004
(73) Patentinhaber: BASF AKTIENGESELLSCHAFT, 67056 Ludwigshafen (DE)
(72) Erfinder: NOACK, Rainer, 04932 Grossthiemig (DE); SANDER, Michael, 67117 Limburgerhof (DE); KAISER, Reinhard, 01984 Meuro (DE)
(86) Internationale Anmeldenummer: PCT/EP2002/005261
(87) Internationale Veröffentlichungsnummer: WO 2002/094818

(56) Entgegenhaltungen:
- EP-A- 0 388 871
- DE-A- 3 806 089
- DE-A- 3 936 823

## Beschreibung

Die vorliegende Erfindung betrifft ein Verfahren zur Reinigung von Triazolylmethyl-oxiranen der Formel I, 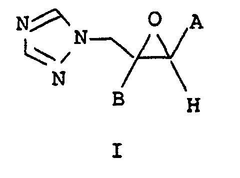 in der A und B gleich oder verschieden sind und unabhängig voneinander C₁-C₄-alkyl, Phenyl-C₁-C₂-alkyl, C₃-C₆-Cycloalkyl, C₃-C₆-Cycloalkenyl, Tetrahydropyranyl, Tetrahydrofuranyl, Dioxanyl oder Phenyl bedeuten, wobei der Phenylrest ein bis drei Substituenten, ausgewählt aus der Gruppe: Halogen, Nitro, C₁-C₄-Alkyl, C₁-C₄-Alkyloxy, Phenoxy, Amino, C₁-C₂-Halogenalkyl oder Phenylsulfonyl tragen kann, die durch Umsetzung eines Oxirans der Formel II 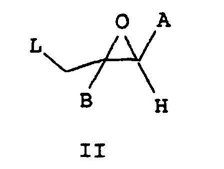 in der A und B die vorgenannte Bedeutung besitzen und L für eine nukleophil austauschbare Abgangsgruppe steht, mit
a) 1,2,4-Triazol (IIIa) und einem Moläquivalent einer anorganischen Base oder
b) 1,2,4-Triazolid der Formel IIIb, 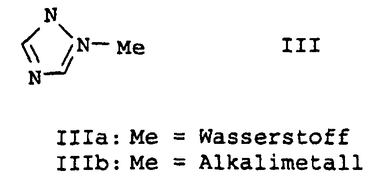 in der Me für ein Alkalimetallatom oder ein quarternäres Ammoniumion steht, in einem aprotischen, dipolaren Lösungsmittel gewonnen werden.

Azolylmethyl-oxirane dienen zur Herstellung von fungiziden Mitteln, insbesondere gegen Getreidekrankheiten.

Aus dem Stand der Technik sind einige Verfahren zur Herstellung von Triazolylmethyl-oxiranen ausgehend von Oxiran II mit Natrium-1,2,4-triazolid bekannt (s. DE-A 39 36 823, EP-A 330132 und EP-A 334 035). Als Lösungsmittel kommen in der Regel Dimethylformamid und N-Methylpyrrolidon zur Anwendung. Die Aufarbeitung der erhaltenen Triazolierungsprodukte erfolgt - wie beispielsweise in der DE-A 39 36 823 beschrieben - durch Fällung mit Wasser und/oder Extraktion.

Die Verfahren des Standes der Technik sind mit einer Reihe von Nachteilen behaftet. Bei der Triazolierung von Verbindungen der Formel II entstehen neben den gewünschten 1- auch 4- substituierte Triazole in Anteilen von 5-25 %.

In Verbindung der Formel I befinden sich darüberhinaus chirale Zentren, so daß im allgemeinen als Diastereomerengemische erhalten werden.

Weiterhin entstehen durch Solvolyse und Ringöffnungsreaktionen eine Reihe von Nebenprodukten, die die Ausbeute verringern und die Isolierung und Reinigung der gewünschten Triazolylmethyloxirane erheblich erschweren.

Zur Reinigung der anfallenden Isomerengemische werden genannt:
- Extraktion (z.B. DE-A 32 18 130, US 4,906,652),
- Fällung (z.B. DE-A 39 36 823),
- Chromatografie (z.B. DE-A 38 06 089),
- Rekristallisation aus Disopropypether (DE-A 39 36 823, US 4,906 652), Methyltert.butylether/n-Hexan (DE-A 38 05 376, EP-A 330 132), Methyltert.butylether (DE-A 37 37 888),

Um die nötige Reinheit der Pflanzenschutzwirkstoffe zu erhalten, muß in der Regel eine Kombination der o.g. Methoden angewandt werden.

Die Reinheit der fungizid wirksamen Isomeren ist überwiegend unter 92 %, nur nach vorbeschriebener komplizierter Aufarbeitung kann man vertretbare Gehalte von über 94 % erreichen.

Wirtschaftliches Verfahren zur Reinigung von Triazolylmethyl-oxiranen der Formel II bis zu Wirkstoffgehalten von über 96 % bei hohen Raum-Zeit-Ausbeuten sind aus den sogenannten Gründen im technischen Maßstab bislang nur beschränkt verfügbar.

Der Erfindung lag deshalb die Aufgabe zugrunde, eine wirtschaftliche Reinigungsmethode für fungizid wirksame Isomere von Azolylmethyl-oxiranen mit dem Ziel hohe Reinheit, hohe Raum-Zeit-Ausbeuten und einfacher Produktisolierung zu finden.

Überraschenderweise konnte ein solches Verfahren zur Reinigung von Triazolyloxiranen dadurch gefunden werden, daß man nach beendeter Reaktion die Reaktionslösung mit einem mit Wasser mischbaren Cosolvenz versetzt and die Triazolylmethyloxiran Isomere anschließend fraktioniert mit Wasser fällt. Demgegenüber wird in den Verfahren nach dem Stand der Technik am Ende der Reaktion die Reaktionsmischung direkt mit Wasser versetzt.

Durch die Anwendung der erfindungsgemäßen Methode verbleiben die Nebenprodukte und die fungizid nicht oder weniger aktiven isomeren Azolylmethyl-oxiranen in Lösung, während die gewünschten aktiven Isomere in hoher Reinheit und Ausbeuteverhalten erhalten werden können.

In der Triazolierungsreaktion werden dipolar aprotischen Lösungsmittel, wie Dimethylformamid, Dimethylacetamid, N-Methylpyrrolidon, Tetramethylharnstoff, Dimethylsulfoxid bzw. Sulfolan verwendet.

Erfindungsgemäß wird dem Reaktionsgemisch am Ende der Reaktion ein mit Wasser mischbares Cosolvens zugesetzt: z.B. niedrigmolekularen Alkoholen wie Methanol, Ethanol, Isopropanol, tert.Butanol, Glykol, Methylglykol etc., Ketonen wie Aceton, Methylethylketon, acycl . und cycl. Ethern wie Dimethoxyethan, Tetrahydrofuran, Dioxan, cycl.Estern wie Glykolcarbonat oder Butyrolacton, Nitrilen wie Acetonitril, Amide wie Formamid,N-Methylformamid, Pyrrolidon etc.

Im folgenden wird das erfindungsgemäße Verfahren näher erläutert.

Für das erfindungsgemäße Verfahren eignen sich Azolylmethyloxiranen, die aus folgenden Ausgangsmaterialien hergestellt worden sind.
a) Oxirane der Formel II in der A und B gleich oder verschieden sind und unabhängig voneinander C₁-C₄-Alkyl, Phenyl-C₁-C₂-alkyl, C₃-C₆-Cycloalkyl, C₃-C₆-Cycloalkenyl, Tetrahydropyranyl, Tetrahydrofuranyl, Dioxanyl oder Phenyl bedeuten, wobei der Phenylrest ein bis drei Substituenten. Ausgewählt aus der Gruppe: Halogen, Nitro, C₁-C₄-Alkyl, C₁-C₄-Alkyloxy, Phenoxy, Amino, C₁-C₂-Halogenalkyl oder Phenylsulfonyl tragen kann und L für eine nukleophil substituierbare Abgangsgruppe steht. Die Oxirane lassen sich wie in EP-A 94 564, US 4,906,652, EP-A 330132, EP-A 334 035 und DE-39 36 823 beschrieben herstellen. Bevorzugte Ausgangsmaterialien tragen die folgenden Substituenten, wobei die Bevorzugung jeweils für sich allein oder in Kombination zu sehen ist:
   A und B bedeuten vorzugsweise einen durch Halogen, C₁-C₄-Alkyl oder C₁-C₄-Alkyloxy substituierten Phenylrest.
   Insbesondere bedeutet A 4-Fluorphenyl und B 2-Chlorphenyl.
   L steht für eine nukleophil substituierbare Abgangsgruppe wie beispielsweise Halogenid, Alkylsulfonat, Arylsulfonat oder Alkylsulfat. Vorzugsweise bedeutet L Chlorid, Bromid, Tosylat und insbesondere Mesylat.

Als Triazolide eignen sich Verbindungen der Formel IIIb, 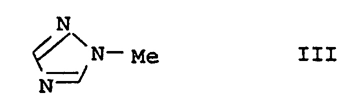 in der Me für ein Alkalimetall oder ein quartäres Ammonium, insbesondere für Natrium oder Kalium steht.

Geeignete Basen zum Einsatz in der Verfahrensvariante a) bzw. Herstellung von Triazolid IIIb (Verfahrensvariante b) sind Alkalihydroxide wie beispielsweise Natrium- oder Kaliumhydroxid, quartäre Ammoniumhydroxide, Alkalimetallcarbonate wie beispielsweise Natrium-, Kalium- oder Natriumhydrogencarbonat, Alkalimetallakoholate wie beispielsweise Natriummethylat oder Kaliumtert.-butylat, Alkalicarboxylat wie beispielsweise Kaliumformylat oder Natriumacetat, Alkalimetallphenolat wie beispielsweise Natriumphenolat, oder Hydride wie Natriumhydrid oder Natriumboranat.

Die bei der Triazolierung erhältliche Reaktionsmischung wird am Ende oder gegebenfalls auch während der Reaktion mit einem Cosovens versetzt. Dabei entsteht eine Lösungsmittelmischung, aus der dann diegewünschten Isomeredurch die fraktionierte Zugabe von wasser gefällt werden können.

Aprotische Cosolventien können bereits bei der Triazolierungsreaktion zugegen sein. Protische Cosolvents sollten immer nach der Triazolierungsreaktion beigefügt werden, da sie die Bildung von Nebenprodukten und nicht gewünschter Isomere begünstigen.

Der Anteil des Cosolvents sollte zwischen 1 und 30 % liegen, vorzugsweise zwischen 2 und 20 %. Der optimale Anteil hängt vom Gehalt an isomeren Azolylmethyl-oxiranen und der Nebenprodukte ab und muß durch Vorversuche ermittelt werden.

Es ist wesentlich für den Erfolg des erfindungsgemäßen Verfahren, daß die Fällung mit Wasser stufenweise erfolgt, wobei bis zur Fällung der ersten Anteile eine langsame Wasserzugabe erforderlich ist.

Die Fälltemperatur sollte im Bereich von 25-60°C liegen, vorzugsweise zwischen 30 und 50°C.

Das Verhältnis Fällwassermenge zur Lösungsmittelmischung zwischen 0,50 und 2,00 vorzugsweise zwischen 1,00 und 1,50.

Das erfindungsgemäße Verfahren wird durch die folgenden Beispiele erläutert.

### Durchführungsbeispiele

### Beispiel 1

110 g einer Lösung, hergestellt aus 100 g MES/DMF (320,5 g/l MES, MES = cis/trans-2-Methansulfonyloxy-methyl-2-(fluorphenyl)-3-(2-chlorphenyl)-oxiran, cis/trans 5:95), 0,2 g Triazol und 10 g Natriumtriazolid durch 5 h Reaktion bei 70°C, wurden auf 30-35°C gekühlt, mit 20 g Aceton versetzt und mit 100 g Wasser schrittweise gefällt. Der ausgefallene Niederschlag wurde abgesaugt und mit 50 ml Methanol gewaschen.

Man erhielt 21,1 g eines Produktes mit folgenden analytischen Daten (HPLC-Flächen-%):

trans-Epoxiconazol = 98 %, sym.-Epoxiconazol = 1,83 %, cis Epoxiconazol = 0,01 % EPA (2-Hydroxy-methyl-2-(fluorphenyl)-3-(2-chlorphenyl)-oxiran) = 0,07 %.

### Beispiel 2-7

Die Durchführung erfolgte analog Beispiel 1, man ersetzte aber das Aceton durch andere Cosolventien (Tab. 1).

**Tab. 1**

| Beispiel Nr. | Cosolvent | trans | sym | cis | EPA |
|---|---|---|---|---|---|
| 2 | MeOH | 96,9 | 2,14 | 0,42 | 0,32 |
| 3 | ButOH | 97,3 | 2,21 | 0,18 | 0,18 |
| 4 | MeCN | 97,6 | 2,05 | 0,14 | 0,11 |
| 5 | MeOCH₂CH₂OH | 97,3 | 1,87 | 0,16 | 0,14 |
| 6 | Dioxan | 97,8 | 1,82 | 0,05 | 0,15 |
| 7 | MeNHCHO | 97,4 | 2,02 | 0,13 | 0,25 |

### Beispiel 8

In einem 10 m³-Reaktor wurden zu 1236 kg MES (cis-trans 5:95), gelöst in 2664 kg DMF 320 kg Triazol und 750 kg Natriummethanolatlösung hinzugefügt und 5 h bei 75°C gerührt. Nach kompletten Umsatz des MES wurden 300 kg Methanol hinzugegeben und auf 35°C gekühlt. Man fällte mit 820 kg 25°C warmen Wasser mit Zugabegeschwindigkeit von anfangs 300 l/h, später mit 800 l/h. Die erhaltene Kristallmaische wurde an einer Schälzentrifuge von der Mutterlauge getrennt und mit Wasser (ca. 4 l/kg Filterkuchen) gewaschen. Man erhielt 750 kg eines Produktes mit folgenden analytischen Daten:
trans-Epoxiconazol = 97 %, sym.-Epoxiconazol = 2,23 %, cis Epoxiconazol <0,01 % EPA (2-Hydroxy-methyl-2-(fluorphenyl)-3-(2-chlorphenyl)-oxiran) = 0,17 %

### Beispiel 9

In einem 2,5 m³-Reaktor wurden zu 330 kg MES (cis-trans 5:95), gelöst in 675 kg DMF 100 kg Natriumtriazolid und 4 kg Triazol hinzugefügt und 5 h bei 65°C gerührt. Nach kompletten Umsatz des MES wurden 110 kg Methanol hinzugegeben und auf 35°C gekühlt. Man fällte mit 25°C warmen Wasser mit Zugabegeschwindigkeit von anfangs 300 l/h, später mit 800 l/h. Die erhaltene Kristallmaische wurde an einer Schälzentrifuge von der Mutterlauge getrennt und mit Wasser (ca. 4 l/kg Filterkuchen) gewaschen. Man erhielt 215 kg eines Produktes mit folgenden analytischen Daten:
trans-Epoxiconazol = 96,7 %, sym.-Epoxiconazol = 2,21 %, cis Epoxiconazol = 0,58 % EPA (2-Hydroxy-methyl-2-(fluorphenyl)-3-(2-chlorphenyl)-oxiran) = 0,34 %.

### Vergleichsbeispiel nach DE 39 36 823

Die Durchführung erfolgte analog Beispiel 9, man verzichtete aber auf den erfindungsgemäßen Zusatz von Methanol und erhielt ein Produkt mit folgenden analytischen Daten:
trans-Epoxiconazol = 94 %, sym.-Epoxiconazol = 3,20 %, cis Epoxiconazol = 1,42 % EPA (2-Hydroxy-methyl-2-(fluorphenyl)-3-(2-chlorphenyl)-oxiran) = 1,00 %.

### Vergleichsbeispiel nach DE 32 18 130

Die Durchführung erfolgte analog Beispiel 2, man verzichtete aber auf den erfindungsgemäßen Zusatz von Aceton, sondern extrahierte mit Ethylacetat. Man erhielt nach dem Eindampfen ein Produkt mit folgenden analytischen Daten (HPLC-F1.-%):
trans-Epoxiconazol = 80,33 %, sym.-Epoxiconazol = 11,28 %, cis Epoxiconazol = 5,20 % EPA (2-Hydroxy-methyl-2-(fluorphenyl)-3-(2-chlorphenyl)-oxiran) = 1,21 %.

Durch Rekristallisation aus Disopropylether konnte man trans-Epoxiconazol unter Ausbeuteverlustes auf einen Gehalt von 96 % anreichern.

## Patentansprüche

1. Verfahren zur Reinigung von Triazolylmethyloxiran-Isomeren der allgemeinen Formel I 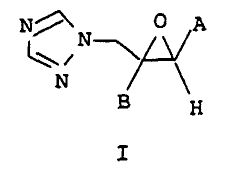 in der A und B gleich oder verschieden sind und unabhängig voneinander C₁-C₄-Alkyl, Phenyl-C₁-C₂-alkyl, C₃-C₆-Cycloalkyl, C₃-C₆-Cycloalkenyl, Tetrahydropyranyl, Tetrahydrofuranyl, Dioxanyl oder Phenyl bedeuten, wobei der Phenylrest ein bis drei Substituenten, ausgewählt aus der Gruppe: Halogen, Nitro, C₁-C₄-Alkyl, C₁-C₄-Alkyloxy, Phenoxy, Amino, C₁-C₂-Halogenalkyl oder Phenylsulfonyl tragen kann, die durch Umsetzung eines Oxirans der Formel II 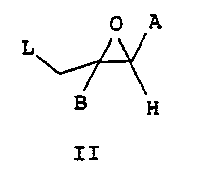 in der A und B die vorgenannte Bedeutung besitzen und L für eine nukleophil austauschbare Abgangsgruppe steht, mit
a) 1,2,4-Triazol (IIIa) und einem Moläquivalent einer anorganischen Base oder
b) 1,2,4-Triazolid der Formel IIIb, 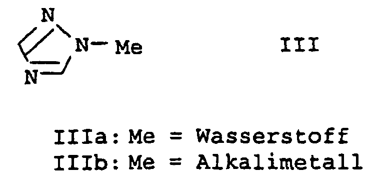 in der Me für ein Alkalimetallatom oder ein quarternäres Ammoniumion steht, in einem aprotischen, dipolaren Lösungsmittel gewonnen werden, **dadurch gekennzeichnet, daß** man nach beendeter Reaktion die Reaktionslösung mit einem mit Wasser mischbaren Cosolvenz versetzt and die Triazolylmethyloxiran Isomere anschließend fraktioniert mit Wasser fällt.

2. Verfahren gemäß Anspruch 1, **dadurch gekennzeichnet, daß** das Cosolvenz ein Lösungsmittel ausgewählt aus der Klasse:
C₁-C₆-Alkohol, Glykol, cyclischer Ether, Nitril, Keton oder Monoalkylformamid oder Pyrrolidon ist.

3. Verfahren gemäß Anspruch 2, **dadurch gekennzeichnet, daß** als Cosolvenz ein C₁-C₄-Alkohol dient.

4. Verfahren gemäß Anspruch 1 bis 3, **dadurch gekennzeichnet, dass** die fraktionierte Fällung durch stufenweise Zugabe von Wasser bei Temperaturen zwischen 25 und 60°C durchgeführt wird.

5. Verfahren gemäß Anspruch 1 bis 4, **dadurch gekennzeichnet, dass** das Verhältnis von Fällwassermenge zu Lösungsmittelmischung zwischen 0,5 und 2 beträügt.

6. Verfahren gemäß Anspruch 1 bis 5, **dadurch gekennzeichnet, daß** in Formel I die Substituenten A = 4-Fluorphenyl und B = 2-Chlorphenyl bedeuten.

## Claims

1. A process for the purification of triazolylmethyloxirane isomers of the formula I 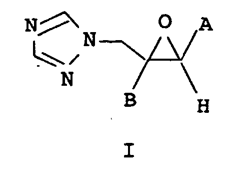 in which A and B are identical or different and, independently of one another, are C₁-C₄-alkyl, phenyl-C₁-C₂-alkyl, C₃-C₆-cycloalkyl, C₃-C₆-cycloalkenyl, tetrahydropyranyl, tetrahydrofuranyl, dioxanyl or phenyl, where the phenyl radical may carry one to three substituents chosen from the group: halogen, nitro, C₁-C₄-alkyl, C₁-C₄-alkyloxy, phenoxy, amino, C₁-C₂-haloalkyl or phenylsulfonyl, which are obtained by reacting an oxirane of the formula II 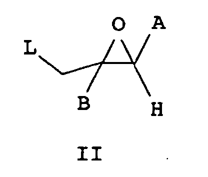 in which A and B have the meanings given above and L is a nucleophilically substitutable leaving group, with
a) 1,2,4-triazole (IIIa) and one mole equivalent of an inorganic base or
b) 1,2,4-triazolide of the formula IIIb, 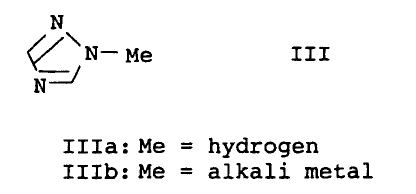
in which Me is an alkali metal atom or a quaternary ammonium ion, in an aprotic dipolar solvent, **characterized in that**, after the reaction has ended, the reaction solution is admixed with a water-miscible cosolvent and then the triazolylmethyloxirane isomers are fractionally precipitated with water.

2. A process according to claim 1, **characterized in that** the cosolvent is a solvent selected from the class:
C₁-C₆-alcohol, glycol, cyclic ether, nitrile, ketone or monoalkylformamide or pyrrolidone.

3. A process according to claim 2, **characterized in that** a C₁-C₄-alcohol serves as cosolvent.

4. A process according to any of claims 1 to 3, **characterized in that** the fractional precipitation is effected by stepwise addition of water at temperatures between 25 and 60°C.

5. A process according to any of claims 1 to 4, **characterized in that** the ratio of precipitating water quantity to solvent mixture is between 0.5 and 2.

6. A process according to any of claims 1 to 5, **characterized in that** the substituents A and B in the formula I are respectively 4-fluorophenyl and 2-chlorophenyl.

## Revendications

1. Procédé de purification d'isomères de triazolylméthyloxiranne de la formule générale I : 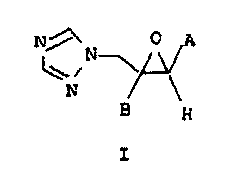 dans laquelle A et B sont identiques ou différents et représentent, indépendamment l'un de l'autre, un radical alkyle en C₁-C₄, phényl-alkyle en C₁-C₂, cycloalkyle en C₃-C₆, cycloalcényle en C₃-C₆, tétrahydropyrannyle, tétrahydrofurannyle, dioxannyle ou phényle, le radical phényle pouvant porter un à trois substituants, choisis parmi le groupe : halogène, nitro, alkyle en C₁-C₄, alkyloxy en C₁-C₄, phénoxy, amino, halogénoalkyle en C₁-C₂ ou phénylsulfonyle, qui sont obtenus par réaction d'un oxiranne de la formule II : 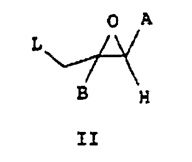 dans laquelle A et B ont la signification donnée ci-dessus et L représente un groupe partant échangeable de manière nucléophile, avec
a) de la 1,2,4-triazole (IIIa) et un équivalent molaire d'une base inorganique, ou
b) du 1,2,4-triazolide de la formule IIIb, 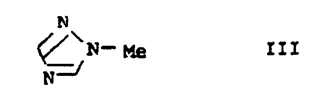 IIIa: Me = hydrogène
IIIb: Me = métal alcalin
où Me représente un atome de métal alcalin ou un ion ammonium quaternaire, dans un solvant dipolaire, aprotique, **caractérisé en ce que**, après achèvement de la réaction, on ajoute à la solution réactionnelle un cosolvant miscible à l'eau et on effectue ensuite une précipitation fractionnée avec de l'eau des isomères de triazolylméthyloxiranne.

2. Procédé suivant la revendication 1, **caractérisé en ce que** le cosolvant est un solvant choisi parmi la classe :
alcool en C₁-C₆, glycol, éther cyclique, nitrile, cétone ou monoalkylformamide ou pyrrolidone.

3. Procédé suivant la revendication 2, **caractérisé en ce qu'**un alcool en C₁-C₄ sert de cosolvant.

4. Procédé suivant l'une des revendications 1 à 3, **caractérisé en ce que** la précipitation fractionnée est effectuée par addition graduelle d'eau à des températures comprises entre 25 et 60°C.

5. Procédé suivant l'une des revendications 1 à 4, **caractérisé en ce que** le rapport entre la quantité d'eau de précipitation et le mélange de solvants est compris entre 0,5 et 2.

6. Procédé suivant l'une des revendications 1 à 5, **caractérisé en ce que**, dans la formule I, le substituant A représente du 4-fluorophényle et le substituant B du 2-chlorophényle.
